Europäisches Patentamt

European Patent Office

Office européen des brevets

(19)

(11) Veröffentlichungsnummer : **0 092 103**
**B1**

(12) # EUROPÄISCHE PATENTSCHRIFT

(45) Veröffentlichungstag der Patentschrift :
28.08.85

(21) Anmeldenummer : 83103333.7

(22) Anmeldetag : 06.04.83

(51) Int. Cl.⁴ : **C 07 C 85/24, C 07 C 85/26**

(54) Verfahren zur Herstellung von o-Toluidin und/oder m-Toluidin und/oder p-Toluidin.

(30) Priorität : 15.04.82 DE 3213876

(43) Veröffentlichungstag der Anmeldung :
26.10.83 Patentblatt 83/43

(45) Bekanntmachung des Hinweises auf die Patenterteilung : 28.08.85 Patentblatt 85/35

(84) Benannte Vertragsstaaten :
CH DE FR GB IT LI NL

(56) Entgegenhaltungen :
EP-A- 0 012 570
US-A- 3 069 470
CHEMICAL ABSTRACTS, Band 92, Nr. 25, 23. Juni 1980, Seite 587, Nr. 215042a, Columbus, Ohio, USA

(73) Patentinhaber : HOECHST AKTIENGESELLSCHAFT
Postfach 80 03 20
D-6230 Frankfurt am Main 80 (DE)

(72) Erfinder : Arpe, Hans-Jürgen, Prof.Dr.
de-Ridder-Weg 10
D-6230 Frankfurt am Main 80 (DE)
Erfinder : Litterer, Heinz, Dr.
Albert-Schweitzer-Allee 39
D-6200 Wiesbaden (DE)

# 0 092 103

## Beschreibung

Die vorliegende Erfindung betrifft ein Verfahren zur Herstellung jedes einzelnen der drei möglichen toluidin-Isomeren, ausgehend von einem der oder dem nicht gewünschten Isomeren bzw. von einem beliebigen Gemisch aus zwei oder allen drei Toluidin-Iosmeren, insbesondere von einem Gemisch, welches das gewünschte Isomere in wirtschaftlich unbefriedigend niedrigen konzentrationen enthält.

o-, m- und p-Toluidin (Methylaniline, Aminotoluole, Toluolamine), d. h. $C_6H_4CH_3NH_2$-Isomere werden technisch zumeist durch Reduktion der entsprechenden Nitrotoluole hergestellt. o-, m- und p-Nitrotoluol selbst entstehen nebeneinander bei der Nitrierung von Toluol. So erhält man beispielsweise unter üblichen Nitrierbedingungen ein Gemisch von 63 % o-, 33-34 % p- und 3-4 % m-Nitrotoluol, das durch eine Kombination von Destillation und Kristallisation mit einer speziellen Methodik, des sog. Abtropfens, in die Reinprodukte zerlegt werden kann (Winnacker-Küchler, Carl Hanser Verlag München 1972, Bd. 4, S. 156). Das Verhältnis der Isomeren ist durch verschiedenen Maßnahmen beeinflußbar (Kirk-Othmer, John Wiley, 3. Aufl. Vol. 15, S. 929) ; so kann beispielsweise der Gewichtsanteil des p-Nitrotoluols zwischen ca. 63-38 % und der des o-Nitrotoluols zwischen 34-58 % schwanken. Dagegen kann der Anteil des m-Nitrotoluols nur in sehr begrenztem Maß, nämlich zwischen 2.3 und 4.3 Gew.-% der gesamt-Nitrotoluol-Ausbeute variiert werden. Damit steht besonders das m-Nitrotoluol für die weitere Reduktion zu m-Toluidin nur in begrenztem Maß sur Verfügung. In keinem Fall gelingt es, durch geeignete Wahl des Katalysators oder der Reaktionsbedingungen zu einem enheitlichen Nitrotoluol-Isomeren zu gelangen. Dieser Tatsache überlagert sich ein unterschiedlicher Marktbdarf sowohl für die Nitrotoluole (als die bevorzugten Ausgangskomponenten für die Toluidine) wie auch für die Toluidine selbst.

Es besteht daher für die unterschiedlichsten Einsatzgebiete der drei Toluidin-Isomeren, wie Herstellung von Farbstoffen, Vulkanisationsbeschleunigern, Textilhilfsmitteln, ein marktabhängiger und damit wechselnder Bedarf, der sich wirtschaftlich nicht optimal, d. h. frei von (zumindest zeitweise) unerwünschten und nicht absetzbaren Nebenprodukten decken läßt.

Der Erfindung lag daher die Aufgabe zugrunde, ein Verfahren zur Herstellung jedes einzelnen der Toluidin-Isomeren zu finden und dabei möglichst von jedem der drei Nitrotoluol-Isomeren als den gängigen Vorstufen der Toluidine auszugehen, so wie es sich in Abhängigkeit vom Marktbedarf für Nitrotoluol-Isomere von Fall zu Fall unterschiedlich als vorteilhaft ergeben kann. Besonders interessant ist dabei der Fall, direkt von Nitriergemischen des Toluols auszugehen, wenn diese eigens zum Zweck der weiteren Hydrierung zu Toluidinen hergestellt wurden. Damit würde dann die zusätzliche und aufwendige, bisher erforderliche Vortrennung der Nitrotoluole durch Destillation und Kristallisation eingespart werden können.

Die Trennung von Toluidin isomeren durch selektive Adsorption an mittel- oder großporigen Zeolithen ist bereits bekannt aus US-A-3069470 und C.A. 92, 2150420. Das erfindungsgemäße Verfahren zur Herstellung von o-Toluidin und/oder m-Toluidin und/oder p-Toluidin unter Verwendnun einer selektiven Adsorption an einem mittel- oder großporigen Zeolithen ist gekennzeichnet durch die beiden folgenden Stufen a und b :

a) Behandlung eines Toluidinisomerengemisches oder des nicht gewünschten oder eines der nicht gewünschten Toluidinisomeren mit einem Isomerisierungskatalysator aus der Reihe der synthetischen Zeolithe vom Pentasil-Typ ;

b) Isolierung des gewünschten oder eines der gewünschten Isomeren aus dem in Stufe a gebildeten Isomerengemisch durch selektive Adsorption an einem mittel- oder großporigen Zeolithen und anschließende Desorption, sowie Ausschleusung des isolierten Isomeren und Rückführung des verbleibenden Isomerengemisches in Stufe a.

Bei geeigneter Zusammensetzung des Toluidinisomerengemisches, d. h. bei einem ausreichenden Gehalt an dem gewünschten Isomeren ist es auch oft vorteilhaft, zuerst eine Adsorption/Desorption zur Abtrennung des bereits im Ausgangsgemisch enthaltenen Anteils am gewünschten Isomeren vorzunehmen und erst anschließend eine Isomerisierung durchzuführen.

Nach dem erfindungsgemäßen Verfahren kann man also o-Toluidin und/oder m-Toluidin und/oder p-Toluidin (die im folgenden oft abkürzend als « o », « m » bzw. « p » bezeichnet werden) herstellen, wobei der Ausdruck « und/oder » nicht die Herstellung von Isomerengemischen andeuten soll, sondern die Tatsache, daß man je nach Wunsch entweder nur eines der Isomeren oder aber zwei (von einander getrennte) Isomere oder aber alle drei (von einander getrennten) Isomeren herstellen kann.

Wenn man nur ein Isomeres herstellen will, kann man von jedem der zwei anderen einzelnen Isomeren ausgehen (z. B. Herstellung von o aus m oder aus p) oder von einem bebliebigen Gemisch aus zwei oder allen drei Isomeren (z. B. Herstellung von o aus o/m, o/p, m/p oder o/m/p).

Wenn man zwei (getrennte) Isomere herstellen will, kann man vom dritten Isomeren ausgehen (z. B. Herstellung von o aus p und gleichzeitig von m aus p) oder von einem beliebigen Gemisch aus zwei oder allen drei Isomeren.

Mann kann aber auch an Stelle des dritten, d. h. nicht gewünschten Isomeren, eines der beiden gewünschten Isomeren nehmen und nur einen Teil davon erfindungsgemäß in das andere gewünschte Isomere überführen.

Wenn man alle drei (getrennten) Isomeren herstellen will, kann man von einem Gemisch aus zwei oder allen drei Isomeren ausgehen. Man kann aber natürlich auch eines der Isomeren nehmen und einen Teil davon erfindungsgemäß in die beiden anderen Isomeren überführen.

Besonders wichtig als Ausgangsmaterial ist das durch Nitrierung und anschließende Hydrierung von Toluol erhaltene Gemisch der drei Toluidine.

Insgesamt kann man also erfindungsgemäß ein beliebiges einzelnes, zwei beliebige oder alle drei Isomeren herstellen, so daß sie am Ende getrennt vorliegen. Das heißt, man kann eine beliebige gewünschte prozentuale Verteilung der drei Isomeren einstellen : 0-100 % o, 0-100 % m, 0-100 % p.

Isomerisierungen werden üblicherweise in Gegenwart von Friedel-Crafts-Katalysatoren wie z. B. von $AlCl_3$, $BF_3$, $BF_3/HF$, $H_3PO_4$, $SnCl_4$, $FeCl_3$, $SbCl_3$, $TiCl_4$ oder $ZnCl_2$, gegebenenfalls in Kombination mit Protonensäuren durchgeführt. Bei Einsatz dieser Katalysatoren zur Isomerisierung von Toluidinen im üblichen Temperaturbereich bis hin zum Siedepunkt der Toluidinisomeren (Kp 200-204 °C) zeigt sich jedoch eine sehr niedrige Isomerisierungsaktivität. Selbst in Gegenwart von Mineralsäuren, wie z. B. HCL in stöchiometrischem Verhältnis zum eingesetzten Toluidin, ist das sonst isomerisierungsaktive $AlCl_3$ ein ungeeigneter Katalysator. Überraschend war nun die hohe Isomerisierungsaktivität von Zeolithen und speziell von synthetischen Zeolithen des Pentasil-Types wie ZSM-5, ZSM-8 oder ZSM-11, welche beispielsweise nach GB-PS 1 567 948 zugänglich sind, sowie auch von natürlichen oder synthetischen Zeolithen vom Mordenit- oder Faujasit-Typ. Dies ist überraschend, da ans EP-A-12570, Seiten 59-61 bekannt war, daß die verwandte Verbindung 1-Amino-4-isopropylbenzol durch Behandlung mit einem ZSM-5-Zeolithen selektiv in Aminobenzol und Propylen zerlegt, aber keineswegs isomerisiert wird. Das Si/Al-Verhältnis der Pentasile liegt vorzugsweise bei etwa 25 bis 2000, das der Mordenite bei vorzugsweise 5 bis 100. Bei Pentasilen oder Mordeniten mit einem höheren Aluminiumgehalt kann eine « Modifizierung » durch Behandlung mit Mineralsäuren, organischen Säuren oder chelatisierenden Substanzen eine teilweise Entfernung des Gerüstaluminiums bewirken, was eine Steigerung der Aktivität zur Folge hat.

Die genannten Zeolithe werden für den technischen Einsatz mit Hilfe von Bindern in Strangform gebracht, wobei die Wahl des Binders die Selektivität und Lebensdauer beeinflußt. Als Bindermaterialien sind vor allem die Oxide, Hydroxide oder Hydroxychloride des Aluminiums und die Oxide bzw. Hydroxide des Siliciums sowie Tone und Claymaterialien geeignet.

Vorzugsweise werden die Zeolithe in der Weise modifiziert, daß man sie durch Ionenaustausch in eine katalytisch besonders aktive Form überführt. Im allgemeinen verwendet man hierzu ein-, zwei- oder dreiwertige Kationen, vorzugsweise $Na^\oplus$, $H^\oplus$, $NH_4^\oplus$, $Be^{2\oplus}$, $Mg^{2\oplus}$, $Ca^{2\oplus}$, die Selten-Erdmetallionen, sowie Kombinationen dieser Elemente.

Die Zeolith-Katalysatoren werden außerdem vorzugsweise in der üblichen Art durch Calcinieren aktiviert, was eine weitere Art der Modifikation darstellt. Gelegentlich ist es vorteilhaft, Ionenaustausch und Calcinierung mehrmals zu wiederholen. Die Calcinierung wird vorzugsweise bei 350 bis 700 °C durchgeführt. Zur besseren Stabilisierung ist es manchmal vorteilhaft, die Calcinierung in Gegenwart von Wasserdampf, Ammoniak oder deren Mischungen bei Temperaturen zwischen 600 und 900 °C durchzuführen.

Die erfindungsgemäße Isomerisierung wird vorzugsweise in der Gasphase mit fest angeordnetem Katalysator durchgeführt. Die Temperaturen betragen dabei im allgemeinen 250 bis 500 °C, vorzugsweise 300-450 °C. Die Umsetzung kann sowohl bei Unterdruck als auch bei Überdruck bis 50 bar vorgenommen werden, vorzugsweise arbeitet man aber bei Normaldruck. Auch andere Verfahrensweisen, wie beispielsweise eine Rieselphase bei Anwendung eines höheren Druckes sind denkbar.

Zusätzlich zu den Toluidinen können zur Herabsetzung des Partialdruckes ggf inerte Löse- oder Verdünnungsmittel über den Katalysator geführt werden. Auch inerte Gase sind zu diesem Zweck geeignet. Man kann auch zusätzlich Wasserstoff gemeinsam mit den Toluidinen über den Katalysator leiten, um gegebenenfalls eine zu rasche Desaktivierung zu vermeiden. Das Molverhältnis von Wasserstoff zu Toluidin kann z. B. zwischen 0,1 : 1 und 10 : 1 liegen.

Wird eine Regenerierung des Katalysators erforderlich, so kann sie im allgemeinen durch kontrolliertes Abrennen mit sauerstoffhaltigen Gasen erfolgen. Die Isomerisierung verläuft nach dem erfindungsgemäßen Verfahren sehr selektiv, sie ergibt in Abhängigkeit von den Prozeßbedingungen, wie z. B. Temperatur und Verweilzeit, sowie abhängig vom Zeolithtyp und vom eingesetzten Toluidin-Isomeren bzw. -Isomerengemisch eine unterschiedliche Zusammensetzung des Reaktionsproduktes ; es bilden sich im allgemeinen hohe Anteile der durch ortho-Verschiebung der Methylgruppe entstehenden Isomeren. So liefert beispielsweise o-Toluidin im Reaktionsgemisch hohe Anteile an m-Toluidin, dagegen weniger p-Toluidin. Setzt man dagegen p-Toluidin in die Isomerisierung ein, so erhält man ebenfalls einen größeren Teil an m-Toluidin und geringe Mengen an o-Toluidin. Die Aufarbeitung des Isomerengemisches kann jedoch wegen der nahezu identischen Siedepunkte von o-Toluidin (Kp 200-202 °C) und p-Toluidin (Kp 200 °C) und des nur wenig höher liegenden Siedepunktes von m-Toluidin (Kp 204 °C) nicht destillativ erfolgen.

Nach dem erfindungsgemäßen Verfahren trennt man die Toluidine adsorptiv an Zeolithen, die je nach zu adsorbierendem Isomeren unterschiedlich ionenausgetauscht sind. Als Zeolithe sind je nach Anforderung X- oder Y-Typen geeignet, die auf den zum Austausch verfügbaren ionischen Stellen die geeigneten Kationen enthalten, die eine Trennung des gewünschten Isomeren vom Isomerengemisch erlauben. Das gewünschte adsorbierte Isomere kann dann anschließend desorbiert werden, wobei auch eine zusätzliche organische Verbindung als Hilfsmittel für die Desorption eingesetzt werden kann.

Wurde zunächst ein gewünschtes Isomeres abgetrennt, so ist die Isolierung eines weiteren Isomeren aus dem verbleibenden Gemisch der zwei anderen Isomeren an einem Zeolith aus der Reihe der X- oder Y-Typen, jedoch mit anderen Kationen als bei der Abtrennung des ersten Isomeren, möglich. Man kann aber auch das besagte Gemisch der beiden Isomeren erneut in die Isomerisierung der Stufe a zurückführen, nämlich wenn von den drei Isomeren nur ein einziges gewonnen werden soll.

In diesem Fall wird durch eine geeignete Wiederholung der Schritte a und b schließlich das Gemisch der drei Isomeren vollständig in nur ein einziges Isomeres übergeführt. Die Entscheidung für den einen oder den anderen Fall hängt ausschließlich von der beabsichtigen technischen Weiterverwendung der Toluidine als Zwischenprodukte für die obengenannten Anwendungsbereiche ab.

Damit ist es als besonderer Vorteil des erfindungsgemäßen Verfahrens hervorzuheben, daß es erstmals möglich geworden ist, Toluol über die Zwischenstufe der drei Nitrotoluole praktisch vollständig in jedes gewünschte reine Toluidin-Isomere überzuführen.

Nach den bisher üblichen Verfahren war durch die wenig variierbare Mischung der drei Nitrotoluol-Isomeren auch gleichzeitig der relative Anteil der Toluidin-Isomeren in engen Grenzen festgelegt.

Die nachfolgenden Beispiele sollen die Erfindung näher erläutern.

## Beispiel 1

ZSM-5, ein synthetischer Zeolith vom Pentasil-Typ, wurde nach bekannten Verfahren, z. B. nach GB-PS 1 567 948 hergestellt und unter Zusatz von 20 Gew.-% $Al_2O_3$ als Bindemittel in Form von Strangpreßlingen extrudiert, bei 120 °C für 12 Stunden getrocknet und bei Temperaturen von 400-500 °C für 4 Stunden calciniert. Anschließend wurde bei etwa 100 °C unter Rückflußbedingungen ein Ionenaustausch mit wäßriger 2n $NH_4NO_3$-Lösung vorgenommen und danach nitrationen-frei gewaschen. Nach Trocknung im Vakuumtrockenschrank bei ca. 100 °C wurde der $NH_4$-ZSM-5 durch fünfstündiges Calcinieren bei 500-550 °C in die Protonenform übergeführt. 50 ml (etwa 27g) des Katalysators in Form von Strangpreßlingen (5-10 mm × 1 mm) wurden in einem Glasreaktor in einer etwa 190 mm hohen Schüttung bei 20 mm Durchmesser angeordnet und durch einen elektrischen Ofen beheizt.

Aus einem beheizten Tropftrichter wurden pro Stunde 10 ml (9,9 g = 92,5 mmol) o-Toluidin zusammen mit 0,5 l/Std. Stickstoff dosiert. Das Isomerisierungsgemisch wurde kondensiert und gaschromatographisch analysiert.

Bei 350 °C Reaktionstemperatur bestand das Isomerengemisch nach 5-6 Stunden Reaktionsdauer aus 67,3 mol-% o-Toluidin, 26,3 mol-% m-Toluidin und 6,4 mol-% p-Toluidin. Nach Temperaturerhöhung auf 400 °C und einer Gesamtreaktionsdauer von 48-56 Std. war die Zusammensetzung des Isomerengemisches 69,3 mol-% o-Toluidin, 22,9 mol-% m-Toluidin und 7,7 mol-% p-Toluidin. Nach insgesamt 88 Std. Reaktionsdauer sind die entsprechenden mol-% Werte für die Toluidin-Isomeren 81,3, 13,9 bzw. 4,8. Der Katalysator erhielt durch kontrolliertes Abbrennen mit sauerstoffhaltigen Gasen wieder seine Ausgangsaktivität zurück.

## Beispiel 2

Ein ZSM-5-Katalysator wurde wie im Beispiel 1 hergestellt, jedoch jetzt (anstelle eines Austausches mit $NH_4NO_3$) durch Behandlung mit 2nHCL für 24 Stunden bei 50-100 °C in die H-ZSM-5-Form übergeführt. Nach dem Waschen mit $H_2O$ bis zur Chlorionen-Abwesenheit wurde der Zeolith bei 100 °C im Trockenschrank getrocknet und bei 400-450 °C 5 Stunden calciniert.

Analog der Versuchsdurchführung im Beispiel 1 wurde eine o-Toluidin-Isomerisierung bei 400 °C durchgeführt. Nach 6 Stunden Reaktionszeit betrug die Zusammensetzung des Toluidingemisches 38,4 mol-% o-, 44,0 mol-% m- und 15,7 mol-% p-Toluidin. Daneben lassen sich geringe Mengen an Anilin nachweisen.

Wurde in analoger Weise p-Toluidin über diesen Katalysator geleitet, so betrug die Isomerenzusammensetzung nach 16 Stunden Reaktionszeit bei 400 °C 18,6 mol-% o-50,4 mol-% m- und 29,2 mol-% p-Toluidin.

Wurde anstelle von p-Toluidin jetzt m-Toluidin über den bereits benutzten Katalysator geleitet, so betrug die Isomerenzusammensetzung nach weiteren 4 Stunden, d. h. nach einer Gesamtreaktionszeit von 20 Stunden, 10,6 mol-% o-, 71,3 mol-% m- und 17,6 mol-% p-Toluidin.

## Beispiele 3-5

ZSM-5-Zeolithe wurden in die Didymium-[1)], Ammonium-, Natrium- bzw. Berylliumform durch Austausch mit den entsprechenden Nitraten übergeführt, gewaschen, getrocknet und calciniert. Die Tabelle zeigt die Ergebnisse für Versuche, bei denen erst 6 Stunden lang o-Toluidin un dann 6 Stunden lang p-Toluidin über denselben Katalysator geleitet wurde. Die Temperatur betrug dabei 400 °C.

(Siehe Tabelle Seite 5 f.)

**0 092 103**

Tabelle

| Katalysator | Ausgangs-material | Kataly-sator-Alter (Std.) | o-Toluidin | m-Toluidin | p-Toluidin |
|---|---|---|---|---|---|
| | | | | ( mol - %) | |
| Di-ZSM-5 | o-Toluidin | 6 | 39.6 | 43.3 | 14.9 |
| " | p-Toluidin | 12 | 10.6 | 52.4 | 33.2 |
| $NH_4$-ZSM-5 | o-Toluidin | 6 | 35.0 | 46.3 | 16.4 |
| " | p-Toluidin | 12 | 6.4 | 46.6 | 42.8 |
| Na-ZSM-5 | o-Toluidin | 6 | 48.7 | 37.9 | 13.3 |
| " | p-Toluidin | 12 | 18.3 | 59.1 | 22.6 |
| Be-ZSM-5 | o-Toluidin | 6 | 46.6 | 38.9 | 14.3 |
| " | p-Toluidin | 12 | 4.1 | 34.6 | 55.5 |

[1] Didymium = handelsübliches Gemisch von Seltenen Erden, bestehend aus Lanthan, Cer, Praseodym, Neodym und kleinen Mengen an Samarium, Gadolinium, Ytterbium u. a.

**Pantetansprüche**

1. Verfahren zur Herstellung von o-Toluidin und/oder m-Toluidin und/oder p-Toluidin, unter Verwendung einer selktiven Adsorption an einem mittel- oder großporigen Zeolithen, gekennzeichnet durch die beiden folgenden Stufen a und b :

a) Behandlung eines Toluidinisomerengemischs oder des nicht gewünschten oder eines der nicht gewünschten Toluidinisomeren mit einem Isomerisierungskatalysator aus der Reihe der synthetischen Zeolithe vom Pentasil-Typ

b) Isolierung des gewünschten oder eines der gewünschten Isomeren aus dem in Stufe a gebildeten Isomerengemisch durch selektive Adsorption an einem mittel- oder großporigen Zeolithen und anschließende Desorption, sowei Ausschleusung des isolierten Isomeren und Rückführung des verbleibenden Isomerengemischs in Stufe a

2. Verfahren nach Anspruch 1, dadurch gekennzeichnet, daß man in Stufe a ein ionenausgetauschtes Pentasil oder einen ionenausgetauschten X- oder Y-Zeolith verwendet.

3. Verfahren nach Anspruch 1 oder 2, dadurch gekennzeichnet, daß man in Stufe b einen ionenausgetauschten X- oder Y-Zeolith verwendet.

4. Verfahren nach Anspruch 2, dadurch gekennzeichnet, daß man als ausgetauschtes Kation $Na^\oplus$, $H^\oplus$, $NH_4^\oplus$, $Be^{2\oplus}$, $Mg^{2\oplus}$, $Ca^{2\oplus}$, die Seltenerdmetall-Ionen oder Kombinationen dieser Kationen verwendet.

5. Verfahren zur Herstellung von o-Toluidin und/oder m-Toluidin und/oder p-Toluidin, gekennzeichnet durch die beiden folgenden Stufen c und d :

c) Isolierung des gewünschten oder eines der gewünschten Isomeren aus einem dieses Isomere in ausreichender Konzentration enthaltenden Toluidin-isomerengemisch durch selektive Adsorption an einem mittel- oder großporigen Zeolithen und anschließende Desorption, sowie Ausschleusung des isolierten Isomeren

d) Behandlung des in Stufe c verbleibenden Isomerengemischs mit einem Isomerisierungskatalysator aus der Reihe der synthetischen Zeolithe vom Pentasil-Typ und Rückführung des gebildeten Isomerengemischs in Stufe c.

6. Verfahren nach Anspruch 5, dadurch gekennzeichnet, daß man in Stufe d ein ionenausgetauschtes Pentasil oder einen ionenausgetauschten X- oder Y-Zeolith verwendet.

7. Verfahren nach Anspruch 5 oder 6, dadurch gekennzeichnet, daß man in Stufe c einen ionenausgetauschten X- oder Y-Zeolith verwendet.

8. Verfahren nach Anspruch 6, dadurch gekennzeichnet, daß man als ausgetauschtes Kation $Na^\oplus$, $H^\oplus$, $NH_4^\oplus$, $Be^{2\oplus}$, $Mg^{2\oplus}$, $Ca^{2\oplus}$, die Seltenerdmetall-Ionen oder Kombinationen dieser Kationen verwendet.

9. Verfahren zur Isomerisierung von o-Toluidin und/oder m-Toluidin und/oder p-Toluidin, gekennzeichnet durch Behandlung eines Toluidinisomerengemischs oder des nicht gewünschten oder eines der nicht gewünschten Toluidinisomeren mit einem Isomerisierungskatalysator aus der Reihe der synthetischen Zeolithe vom Pentasil-Typ.

10. Verfahren nach Anspruch 9, dadurch gekennzeichnet, daß man ein ionenausgetauschtes Pentasil oder einen ionenausgetauschten X- oder Y-Zeolith verwendet.

11. Verfahren nach Anspruch 10, dadurch gekennzeichnet, daß man als ausgetauschtes Kation $Na^\oplus$, $H^\oplus$, $NH_4^\oplus$, $Be^{2\oplus}$, $Mg^{2\oplus}$, $Ca^{2\oplus}$, die Seltenerdmetall-Ionen oder Kombinationen dieser Kationen verwendet.

5

**Claims**

1. A process for the manufacture of o-toluidine and/or m-toluidine and/or p-toluidine involving a selective adsorption to a medium-pore or large-pore zeolite- characterized by the two following steps a) and b) :

a) treatment of a toluidine isomer mixture or of the not intended toluidine isomer or of one of the not intended toluidine isomers with an isomerization catalyst selected from the group of synthetic zeolites of the pentasil type,

b) isolation of the intended isomer or one of the intended isomers form the isomer mixture formed in step a) by selective adsorption to a medium-pore or large-pore zeolite and subsequent desorption, furthermore discharge of the isolated isomer and recycling of the remaining isomer mixture to step a).

2. The process as claimed in Claim 1, characterized by using in step a) a pentasil or X or Y zeolite the ions of which are exchanged in each case.

3. The process as claimed in Claim 1 or 2, characterized by using in step b) and X or Y zeolite the ions of which are exchanged.

4. The process as claimed in Claim 2, characterized by using as exchanged cation $Na^{\oplus}$, $H^{\oplus}$, $NH_4^{\oplus}$, $Be^{2\oplus}$, $Mg^{2\oplus}$, $Ca^{2\oplus}$, rare earth metal ions, or combinations of these cations.

5. A process for the manufacture of o-toluidine and/or m-toluidine and/or p-toluidine, involving a selective adsorption to a medium-pore or large-pore zeolite characterized by the following two steps c) and d) :

c) isolation of the intended isomer or one of the intended isomers from a toluidine isomer mixture containing this isomer in sufficient concentration by selective adsorption to a medium-pore or large-pore zeolite and subsequent desorption, and discharge of the isolated isomer,

d) treatment of the isomer mixture remaining in step c) with an isomerization catalyst selected from the group of synthetic zeolites of the pentasil type, and recycling of the isomer mixture formed to step c).

6. The process as claimed in Claim 5, which comprises using in step d) a pentasil or X or Y zeolite the ions of which are exchanged in each case.

7. The process as claimed in Claim 5 or 6, which comprises using in step C) an X or Y zeolite the ions of which are exchanged.

8. The process as claimed in Claim 6, which comprises using as exchanged cation $Na^{\oplus}$, $H^{\oplus}$, $NH_4^{\oplus}$, $Be^{2\oplus}$, $Mg^{2\oplus}$, $Ca^{2\oplus}$, rare earth metal ions, or combinations of these cations.

9. A process for the isomerisation of o-toluidine and/or m-toluidine and/or p-toluidine, characterized by treatment of a toluidine isomer mixture or of the not intended toluidine isomer or of one of the not intended toluidine isomers with an isomerization catalyst selected from the group of synthetic zeolites of the pentasil type.

10. The process as claimed in Claim 9, characterized by using a pentasil or X or Y zeolite the ions of which are exchanged in each case.

11. The process as claimed in Claim 10, characterized by using as exchanged cation $Na^{\oplus}$, $H^{\oplus}$, $NH_4^{\oplus}$, $Be^{2\oplus}$, $Mg^2$, $Ca^{2\oplus}$, rare earth metal ions, or combinations of these cations.

**Revendications**

1. Procédé de préparation de l'o-toluidine et/ou de la m-toluidine et/ou de la p-toluidine, comprenant une adsorption sélective sur une zéolite à pores moyens ou grands, procédé caractérisé en ce qu'il comporte les deux étapes a et b suivantes :

a) on traite un mélange de toluidines isomères, ou l'isomère de la toluidine non recherché, ou une des toluidines isomères non recherchées, par un catalyseur d'isomérisation pris dans l'ensemble des zéolites synthétiques du type du Pentasil,

b) du mélange d'isomères formé dans l'étape a on isole l'isomère voulu ou un des isomères voulus par adsorption sélective sur une zéolite à pores moyens ou grands, puis on déborde, on fait sortir l'isomère isolé et on renvoie à l'étape a le mélange d'isomères restant.

2. Procédé selon la revendication 1 caractérisé en ce qu'on utilise, dans l'étape a, un Pentasil qui a subi un échange d'ions ou une zéolite X ou Y qui a subi un échange d'ions.

3. Procédé selon l'une des revendications 1 et 2, caractérisé en ce qu'on utilise, dans l'étape b, une zéolite X ou Y qui a subi un échange d'ions.

4. Procédé selon la revendication 2 caractérisé en ce qu'on utilise, comme cations échangés, $Na^{\oplus}$, $H^{\oplus}$, $NH_4^{\oplus}$, $Be^{2\oplus}$, $Mg^{2\oplus}$, $Ca^{2\oplus}$, les ions des terres rares ou des associations de ces cations.

5. Procédé de préparation de l'o-toluidine et/ou de la m-toluidine et/ou de la p-toluidine, comprenant une adsorption sélective sur une zéolite à pores moyens ou grands, procédé caractérisé en ce qu'il comporte les deux étapes c et d suivantes :

c) on isole l'isomère voulu, ou un des isomères voulus, à partir d'un mélange de toluidines isomères contenant cet isomère en une concentration suffisante, par adsorption sélective sur une zéolite à pores moyens ou grands, après quoi on désorbe et on fait sortir l'isomère isolé,

d) on traite le mélange d'isomères restant dans l'étape c par un catalyseur d'isomérisation pris dans l'ensemble des zéolites synthétiques du type du Pentasil, et on renvoie à l'étape c le mélange d'isomères formé.

6. Procédé selon la revendication 5 caractérisé en ce qu'on utilise, dans l'étape d, un Pentasil qui a subi un échange d'ions ou une zéolite X ou Y qui a subi un échange d'ions.

7. Procédé selon l'une des revendications 5 et 6, caractérisé en ce qu'on utilise, dans l'étape c, une zéolite X ou Y qui a subi un échange d'ions.

8. Procédé selon la revendication 6 caractérisé en ce qu'on utilise, comme cations échangés, $Na^{\oplus}$, $H^{\oplus}$, $NH_4^{\oplus}$, $Be^{2\oplus}$, $Ca^{2\oplus}$, les ions des terres rares ou des associations de ces cations.

9. Procédé pour isomériser l'o-toluidine et/ou la m-toluidine et/ou la p-toluidine, caractérisé en ce qu'on traite un mélange de toluidines isomères, ou l'isomère non recherché de la toluidine par un catalyseur d'isomérisation pris dans l'ensemble des zéolites synthétiques du type du Pentasil.

10. Procédé selon la revendication 9 caractérisé en ce qu'on utilise un Pentasil ayant subi un échange d'ions ou une zéolite X ou Y ayant subi un échange d'ions.

11. Procédé selon la revendication 10 caractérisé en ce qu'on utilise, comme cations échangés, $Na^{\oplus}$, $H^{\oplus}$, $NH_4^{\oplus}$, $Be^{2\oplus}$, $Mg^{2\oplus}$, $Ca^{2\oplus}$, les ions des terres rares ou des associations de ces cations.